# FASCICULE DE BREVET EUROPEEN

(11) **EP 3 623 012 B1**
(45) Date de publication et mention de la délivrance du brevet: **29.07.2026**
(21) Numéro de dépôt: 19197166.2
(22) Date de dépôt: 13.09.2019
(51) Int. Cl.: A61K 8/9783, A61Q 1/00, A61Q 19/00, A61Q 19/08

(54) **PRINCIPE ACTIF COSMETIQUE OBTENU A PARTIR DE L'ALBUMEN DU FRUIT DE COCOS NUCIFERA ET UTILISATIONS COSMETIQUES**
KOSMETISCHER WIRKSTOFF AUF DER BASIS VON ALBUMEN DER KOKOSNUSSFRUCHT, UND KOSMETISCHE ANWENDUNGEN
COSMETIC ACTIVE INGREDIENT OBTAINED FROM THE ALBUMEN OF THE FRUIT OF COCOS NUCIFERA AND COSMETIC USES

(30) Priorité: 14.09.2018 FR 1858272
(43) Date de publication de la demande: 18.03.2020
(73) Titulaire: Societe Industrielle Limousine d'Application Biologique, 19240 Saint Viance (FR)
(72) Inventeur: PAUFIQUE, Jean, 19130 OBJAT (FR)
(74) Mandataire: Aquinov

(56) Documents cités:
- WO-A1-2010/127396
- WO-A1-2010/127396
- WO-A1-2012/058722
- WO-A1-2017/158014
- CN-A- 107 550 822
- CN-A- 108 191 961
- CN-B- 104 171 261
- JP-A- H05 331 045
- US-A1- 2011 123 648
- PATIL UMESH ET AL: "Characteristics of albumin and globulin from coconut meat and their role in emulsion stability without and with proteolysis", FOOD HYDROCOLLOIDS, ELSEVIER BV, NL, vol. 69, 17 February 2017 (2017-02-17), pages 220 - 228, XP029976048, ISSN: 0268-005X, DOI: 10.1016/J.FOODHYD.2017.02.006

## Description

La présente invention concerne la cosmétique et en particulier un principe actif cosmétique spécifique obtenu à partir de l'albumen du fruit de *Cocos nucifera.* L'invention a également pour objet l'utilisation cosmétique de ce principe actif en application topique sur la peau pour embellir la peau et notamment la régénérer et augmenter sa sensorialité.

La peau tient le rôle d'interface entre l'organisme et l'environnement externe. Constamment soumise à des stimulations environnementales, elle est pourvue de multiples récepteurs afin de transmettre les informations perçues au cerveau, sous forme d'influx nerveux.

Différents récepteurs ont été décrits au sein de la peau : des mécanorécepteurs, thermorécepteurs, photorécepteurs ainsi que des chémorécepteurs. Des études ont récemment mis en évidence l'expression de nouvelles structures de chémorécepteurs dans la peau : les récepteurs olfactifs et gustatifs. Le rôle de ces récepteurs alliés aux sens va bien au-delà de la détection de molécules chimiques. En effet, l'activation des récepteurs de l'olfaction et du goût module de différentes manières l'homéostasie de la peau. Ces relais de l'information sensorielle influencent la prolifération cellulaire, la migration, la différenciation et favorisent ainsi la régénération du tissu cutané. De plus, le demandeur a mis en place des modèles démontrant que l'expression et le fonctionnement de ces récepteurs étaient dérégulés au cours du vieillissement.

Il est connu de l'art antérieur (JP H05 331045) un produit à base de lait de coco, à savoir une émulsion qui, par définition, n'est pas hydrosoluble, notamment pour hydrater la peau.

L'objectif de l'invention est de proposer un principe actif cosmétique capable de restaurer les récepteurs sensoriels épidermiques, pour augmenter la sensorialité de la peau et la régénérer. A cet effet, l'invention vise un principe actif cosmétique hydrosoluble obtenu à partir de l'albumen du fruit de *Cocos nucifera,* comprenant au moins deux disaccharides lipidiques, ledit principe étant susceptible d'être obtenu par un procédé comprenant les étapes suivantes :
a. solubilisation d'albumen de fruit de *Cocos nucifera* dans un mélange hydroglycolique,
b. séparation des phases soluble et insoluble par décantation,
c. filtrations successives.

Le cocotier, *Cocos nucifera,* aussi nommé Arbre de Vie dans les pays tropicaux, présente des vertus biologiques qui lui confèrent maints usages médicinaux et traditionnels. Les fruits du cocotier murissent au creux de ses palmes verdoyantes, à plusieurs dizaines de mètres de hauteur. Ils sont protégés par une épaisse enveloppe de fibres brunes assurant une flottabilité exceptionnelle aux noix de coco qui peuvent être transportées par les courants marins et s'adapter à des rivages lointains. Ces fibres sont récoltées selon un procédé ancestral afin d'obtenir le coir. Celui-ci est très apprécié dans le tissage et la confection de nombreux objets. Les noix de coco sont également prisées pour leur grande richesse nutritionnelle, ainsi que pour la fragrance fraîche et atypique qu'elles exhalent une fois ouvertes.

En effet, la noix de coco renferme en son cœur une eau opalescente et légèrement sucrée en bouche, connue pour ses propriétés hydratantes et anti-oxydantes. Sa chair blanche et enivrante est composée de nombreux minéraux, vitamines et lipides. C'est une ressource précieuse, à l'origine de différents produits utilisés en cuisine pour leurs flaveurs aussi agréables que caractéristiques. Une première étape de pressage à froid de la chair fraîche permet d'obtenir le lait de coco aux arômes crémeux. Cette pression à froid préserve les arômes et les molécules actives de la matière végétale. Une seconde étape sépare l'huile de coco vierge du lait. Cette huile anti-âge adoucit la peau et la parfume d'une note délicate reconnaissable entre toute. Le co-produit issu de ces procédés est la farine de coco issue de l'albumen de ce fruit.

Avantageusement, l'invention utilise ce co-produit issu de l'albumen du fruit du cocotier pour obtenir un principe actif cosmétique hydrosoluble comprenant au moins deux disaccharides lipidiques, capable de restaurer les récepteurs sensoriels épidermiques et ainsi favoriser la régénération de l'épiderme et garantir le maintien d'une fonction barrière robuste et efficace au fil du temps. Avantageusement, la barrière cutanée est renforcée, la peau est plus lisse, mieux hydratée et plus confortable. En outre, le principe actif cosmétique selon l'invention avec son action pluri-sensorielle procure une sensation de fraîcheur et exalte la sensorialité de la peau.

L'invention a par conséquent également pour objet l'utilisation cosmétique d'un principe actif cosmétique hydrosoluble obtenu à partir de l'albumen du fruit de *Cocos nucifera* comprenant au moins deux disaccharides lipidiques, ou d'une composition en contenant, en application topique sur une peau saine (c'est-à-dire non malade) pour embellir la peau et notamment la régénérer, augmenter sa sensorialité, la lisser et/ou l'hydrater et/ou pour un effet fraicheur.

L'invention vise également les compositions cosmétiques comprenant un principe actif cosmétique selon l'invention, ainsi qu'un procédé cosmétique de traitement de la peau pour un effet régénérant et une augmentation de la sensorialité de la peau, qui consiste en l'application topique sur la peau d'une telle composition.

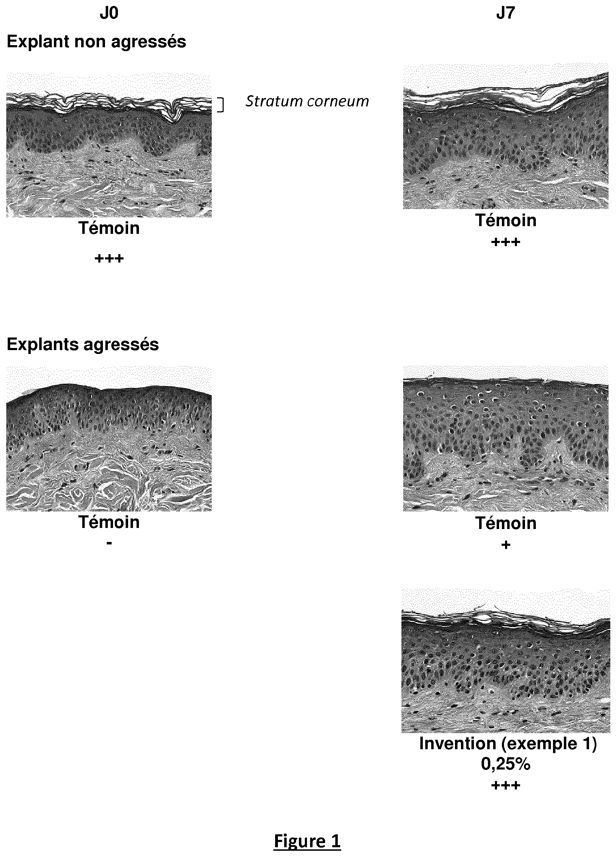

D'autres caractéristiques et avantages ressortiront de la description détaillée de l'invention qui va suivre en regard des exemples et résultats d'essais et de la Figure 1 annexée qui représente des images d'explants de peau humaine obtenues selon le protocole décrit au point III.2 de la partie résultats d'essais, montrant l'effet de l'invention sur la régénération épidermique.

### DEFINITIONS

Par « actif cosmétique » ou « principe actif cosmétique » ou « principe actif » au sens de l'invention, on entend au moins une molécule, préférentiellement un ensemble de molécules présentant un effet cosmétique sur la peau.

Par « principe actif issu de l'albumen du fruit de *Cocos nucifera »* au sens de l'invention, on entend tout extrait issu de l'albumen du fruit de *Cocos nucifera,* albumen entier ou farine de coco, obtenu par un procédé d'extraction.

Par « hydrosoluble » au sens de l'invention, on entend une solution à une concentration au minimum de 50g/L qui se dissout totalement dans l'eau. La dissolution peut être vérifiée au microscope, et forme alors un mélange homogène.

Par « sensorialité » au sens de l'invention on entend la capacité à être réceptif aux sensations (physiques, chimiques, etc). Elle est indispensable à la perception de notre environnement et elle est rendue possible grâce aux organes dits sensoriels dont fait partie la peau.

Par « sensorialité de la peau » au sens de l'invention on entend la capacité à être réceptif aux sensations (physiques, chimiques, etc) par la peau. La peau met en jeu des structures particulières, souvent des récepteurs sensoriels, qui perçoivent les informations tactiles (toucher), olfactives (odorat) et gustatives (goût).

### DESCRIPTION DETAILLEE DE L'INVENTION

L'invention se rapporte donc à un principe actif cosmétique hydrosoluble obtenu à partir de l'albumen du fruit de *Cocos nucifera,* comprenant au moins deux disaccharides lipidiques.

Le principe actif selon l'invention est donc un extrait hydrosoluble obtenu à partir de l'albumen du fruit de *Cocos nucifera* ledit extrait comprenant au moins deux disaccharides lipidiques, ledit principe étant susceptible d'être obtenu par un procédé comprenant les étapes suivantes :
a. solubilisation d'albumen de fruit de *Cocos nucifera* dans un mélange hydroglycolique,
b. séparation des phases soluble et insoluble par décantation,
c. filtrations successives.

Un disaccharide lipidique est un glycolipide particulier. Préférentiellement le principe actif selon l'invention comprend au moins les deux disaccharides lipidiques suivants : le butyl-O-alpha-D-glucopyranosyl-beta-D-glucopyranoside, pouvant être noté marqueur AG-1 et le nonioside D, pouvant être noté marqueur A-1. Les disaccharides lipidiques présents dans le principe actif selon l'invention présentent un effet sur les récepteurs sensoriels de la peau. Préférentiellement, le principe actif selon l'invention comprend au moins 3 ppm de nonioside D et/ou au moins 0,3 ppm de butyl-O-alpha-D-glucopyranosyl-beta-D-glucopyranoside. Etant donné qu'il n'existe pas de standards commerciaux pour les disaccharides lipidiques, il est possible d'utiliser une structure approchante afin d'étalonner et de quantifier les molécules présentes dans le principe actif. Ainsi, il est possible d'utiliser le trehalose 6-docanoate CAS n°924885-56-3 pour estimer la teneur en nonioside D, et le n-decyl-β-D-maltoside CAS n°82494-09-5 pour estimer la teneur en Butyl 4-O-alpha-D-glucopyranosyl-beta-D-glucopyranoside.

En plus des disaccharides lipidiques, le principe actif selon l'invention (l'extrait) peut comprendre d'autres molécules. Il peut notamment comprendre au moins un sucre, tel que par exemple un ou plusieurs sucres choisis parmi les polyols, les polyols carbocycliques, les disaccharides, les tri-saccharides et les tétra-saccharides.

Il peut également comprendre des cendres minérales et/ou des protéines.

Selon un mode de réalisation particulier, le principe actif selon l'invention présente une ou plusieurs des caractéristiques suivantes, préférentiellement toutes :
- une teneur en sucres d'au moins 40% en poids par rapport au poids de matières sèches du principe actif, de préférence au moins 60%,
- les sucres ont des degrés de polymérisation compris entre 2 et 10,
- les sucres sont principalement des disaccharides, des disaccharides lipidiques, des trisaccharides et des tétrasaccharides, des polyols ou des polyols-carbocycliques,
- une teneur en cendres minérales comprise entre 10 et 50% en poids par rapport au poids de matières sèches du principe actif,
- une teneur en protéines inférieure à 10% en poids par rapport au poids de matières sèches du principe actif,
- pas de lipides ou une teneur en lipides inférieure à 1% en poids par rapport au poids de matières sèches du principe actif.

La teneur en sucres dans le principe actif peut être déterminée par la méthode de DUBOIS (Dubois M. et al., Analytical chemistry, 28, 3, 350-356, 1956).

Les carbohydrates peuvent être identifiés par SM/SM et la taille des masses molaires par HPLC/UV.

Les masses molaires des carbohydrates présents dans le principe actif selon l'invention sont préférentiellement les suivantes :

**Tableau 1**

| | Masse molaire (Da) et degré de polymérisation | Taux de carbohydrates (%) |
|---|---|---|
| Monosaccharides | MM ≤ 180 (DP1) | 0 |
| Oligosaccharides et Polysaccharides | 360 < MM ≤ 1800 | 100 |
| | DP2 < DP ≤ 10 | |

Ainsi, préférentiellement, les carbohydrates principe actif selon l'invention sont exclusivement constitués d'oligosaccharides de masse molaire allant de 360Da à 1800Da.

L'identification des espèces principales détectées en MS est préférentiellement la suivante :

**Tableau 2**

| Famille molécule | Nom molécule |
|---|---|
| Disaccharide-lipidique | Nonioside D |
| | Butyl-4-O-alpha-D-glucopyranosyl-beta-D-glucopyranoside |
| Polyol | Sorbitol ou D-mannitol |
| Disaccharide | Saccharose |
| | Mannobiose |
| Polyol carbocyclique | Inositol ou isomère |
| Tri-saccharide | Mannotriose |
| Tétra-saccharide | Stachyose |

La fraction glucidique du principe actif selon l'invention est préférentiellement majoritairement constituée de disaccharides dont certains sont estérifiés par un acide gras. La teneur en cendres minérales peut être déterminée par la pesée des résidus issus de l'incinération des échantillons du principe actif selon l'invention à 550°C dans un four à moufle électrique.

La teneur en protéines peut-être déterminée par la méthode de LOWRY (Lowry et al., Protein measurement with the folin reagent, J. Biol. Chem., 193, 265, 1951) ou par le dosage de l'azote total selon la méthode de KJELDHAL (référence : Official method of analysis of the A.O.C., 12th ed. W Horwitz, E.D., New-York, 15-60, 1975).

De façon préférée, le principe actif selon l'invention se présente sous forme liquide. Le taux de matières sèches du principe actif selon l'invention peut alors être compris entre 10 et 50g/l, préférentiellement entre 20 et 31g/l.

Le principe actif selon l'invention est préférentiellement de couleur jaune très clair. Il peut être éventuellement de couleur jaune plus foncé.

Le principe actif peut être obtenu par tout type de procédé d'extraction permettant d'obtenir au moins deux disaccharides lipidiques, préférentiellement des disaccharides lipidiques choisis au moins parmi le butyl-O-alpha-D-glucopyranosyl-beta-D-glucopyranoside et le nonioside D.

Selon l'invention, le principe actif est obtenu par extraction avec un mélange hydroglycolique de solvants, préférentiellement eau/butylène glycol, de l'albumen du fruit de *Cocos nucifera,* en particulier avec un mélange de solvants constitué par entre 75% et 95% d'eau et par entre 5 et 25% de butylène glycol par la mise en œuvre des étapes suivantes :
- solubilisation d'albumen de fruit de *Cocos nucifera* dans un mélange hydroglycolique,
- séparation des phases soluble et insoluble par décantation,
- Filtrations successives.

Des étapes de désodorisation peuvent aussi être ajoutées.

Le principe actif peut ensuite éventuellement être associé à un support et séché (atomisation ou lyophilisation notamment), pour se présenter sous forme solide.

Les étapes des procédés décrits ci-avant, prises individuellement, sont usuelles dans le domaine des extractions d'actifs à partir de matières premières naturelles et l'homme du métier est à même d'en ajuster les paramètres réactionnels sur la base de ses connaissances générales.

Le principe actif selon l'invention est particulièrement efficace pour un traitement cosmétique, non thérapeutique.

Le principe actif selon l'invention présente une action efficace sur les récepteurs sensoriels épidermiques pour régénérer la peau, augmenter sa sensorialité, l'hydrater, la lisser et/ou pour un effet fraicheur.

L'invention a par conséquent également pour objet l'utilisation cosmétique d'un principe actif cosmétique hydrosoluble issu de l'albumen du fruit de *Cocos nucifera* tel que décrit précédemment ou d'une composition en contenant, en application topique sur la peau saine pour embellir la peau.

Le principe actif selon l'invention peut être utilisé pour augmenter la présence des récepteurs olfactifs épidermiques (en particulier OR2AT4, OR2A4/7 et OR51B5) et rétablir la synthèse des récepteurs gustatifs épidermiques (en particulier TAS2R1 et TAS2R38), et ainsi :
- Augmenter la sensorialité de la peau, en particulier d'apporter un effet fraicheur et une augmentation de la perception de douceur,
- Favoriser la régénération épidermique de la peau notamment en améliorant les capacités migratoires et de différenciation des kératinocytes et en diminuant les pertes insensibles en eau.

L'utilisation sur la peau du principe actif selon l'invention permet ainsi d'hydrater la peau, de la lisser, de la rendre plus douce, plus fraiche et plus confortable.

Le principe actif selon l'invention présente donc une activité biologique cosmétique régénérante, en particulier pour les peaux âgées, tout en en offrant une expérience sensorielle nouvelle.

Initialement décrits dans les tissus olfactifs, les récepteurs responsables de la détection des odeurs ont été identifiés aujourd'hui dans de nouveaux tissus, dont la peau. Il a été démontré récemment que les cellules cutanées font partie intégrante du réseau sensoriel impliqué dans la détection et la transmission des informations olfactives. La perception des odeurs s'effectue *via* des récepteurs olfactifs exprimés à leur surface. Les kératinocytes en particulier représentent de réels relais de l'information sensorielle. Ces senseurs d'odeurs permettent d'établir une véritable communication entre l'épiderme et le système nerveux, berceau de l'émotion et des sensations.

Il est actuellement bien établi qu'au-delà de la détection des odeurs, ces récepteurs possèdent des fonctions dans divers processus biologiques fondamentaux. En effet, leur stimulation par les senteurs favorise la régénération épidermique. Parmi les 350 récepteurs olfactifs (« ORs, Olfactory Receptors ») connus de l'épithélium nasal, plusieurs ont été identifiés dans la peau. Le récepteur OR2AT4, exprimé par les kératinocytes des couches basale et suprabasale de l'épiderme, est un des premiers dont le rôle pro-régénérant a été démontré dans la peau. Même si à ce jour, aucun ligand endogène n'est identifié, OR2AT4 est activé par le bois de santal, bois d'arbre délicieusement parfumé utilisé fréquemment dans les bâtons d'encens et la formulation de parfum. L'exploration a été étendue aux récepteurs OR2A4/7 et OR51B5 qui, de la même manière que OR2AT4, favorisent la régénération épidermique une fois activés par des ligands chimiques.

L'étude de modélisation est exposée dans la partie « Essais et Résultats ». Son objectif est d'appréhender l'évolution de l'expression des récepteurs olfactifs dans la peau avec l'âge, et de montrer la diminution de la présence des récepteurs OR2AT4, OR2A4/7 et OR51B5 dans l'épiderme au cours du vieillissement.

Avantageusement, le principe actif selon l'invention est capable d'augmenter l'expression des récepteurs OR2A4/7 et OR51B5 et de restaurer la synthèse de OR2AT4. Le principe actif selon l'invention permet ainsi sur les peaux saines de maintenir l'expression et la synthèse des récepteurs olfactifs épidermiques au cours du vieillissement.

Par ailleurs, le goût fait partie des nombreuses fonctions sensorielles que possède notre corps. Le goût est indissociable de l'odorat puisque la détection et la distinction des saveurs fait appel à une parfaite symbiose entre ces deux sens.

Les récepteurs du goût ne se limitent pas seulement aux organes gustatifs, leur expression a ainsi été mise en lumière en divers autres endroits du corps. En effet, les récepteurs olfactifs ne détiennent pas le monopôle sensoriel dans la peau puisque la présence de récepteurs gustatifs y a récemment été révélée, et plus précisément au niveau des kératinocytes. Ces deux types de récepteurs se retrouvent donc dans l'épiderme où ils agissent de concert pour une action bénéfique. Parmi les récepteurs gustatifs (TAS2Rs, Taste 2 receptors) existant dans le corps, TAS2R1 et TAS2R38 ont été identifiés dans la peau. Leur expression est croissante de la couche basale de l'épiderme vers le *stratum corneum.* Ces récepteurs sont dotés d'une activité biologique favorisant la différenciation kératinocytaire. Ainsi, en promouvant la synthèse de marqueurs de différenciation précoces et tardifs, les TAS2Rs participent à l'établissement d'une barrière cutanée robuste et à sa régénération.

L'étude de modélisation exposée dans la partie « Essais et Résultats », dont l'objectif est d'appréhender l'évolution de l'expression des récepteurs gustatifs dans la peau avec l'âge, montre une diminution de la présence des récepteurs TAS2R1 et TAS2R38 dans l'épiderme au cours du vieillissement.

Avantageusement, le principe actif selon l'invention est capable d'augmenter l'expression des récepteurs TAS2R1 et TAS2R38. Le principe actif selon l'invention permet ainsi d'augmenter l'expression et la synthèse des récepteurs gustatifs épidermiques au cours du vieillissement. Selon un autre aspect, on sait que les dommages induits dans l'épiderme entraînent l'activation de mécanismes de réparation afin de restaurer rapidement l'intégrité de la barrière cutanée. La régénération épidermique est un processus hautement coordonné faisant intervenir plusieurs activités biologiques dont la ré-épithélialisation, correspondant à la formation d'un nouvel épithélium. Cette étape clé est essentielle au renouvellement efficace du tissu. Au niveau de l'épiderme, les kératinocytes sont les acteurs majeurs de la régénération. Ils prolifèrent, migrent et se différentient pour former un nouvel épiderme robuste. Les récepteurs olfactifs sont décrits pour leurs effets bénéfiques sur la régénération épidermique. En effet, le récepteur olfactif OR2AT4 est capable d'induire la prolifération et la migration kératinocytaires ainsi que la ré-épithélialisation d'explants de peau humaine. De la même manière, OR2A4/7 et OR51B5 jouent des rôles importants dans la prolifération et la migration des kératinocytes, processus indispensables à la régénération épidermique. Si les récepteurs olfactifs favorisent la prolifération et la migration, les études ont démontré que les récepteurs gustatifs, en particulier TAS2R1 et TAS2R38, prennent en charge le volet différenciation kératinocytaire. Cette différenciation est marquée par l'augmentation de l'expression de marqueurs précoces et tardifs tels que la cytokératine 10, l'involucrine ou encore la transglutaminase 1. Ainsi, en promouvant le processus de différenciation, les TAS2Rs participent à l'établissement d'une barrière cutanée robuste et à sa régénération.

Dans la peau, il existe donc une symbiose entre ces deux récepteurs sensoriels qui agissent de concert pour favoriser les mécanismes biologiques inhérents à la régénération épidermique. Les études réalisées dans la partie « Essais et Résultats », dont l'objectif est d'observer l'évolution de la capacité migratoire des kératinocytes avec l'âge, montrent que celle-ci est diminuée.

Avantageusement, l'utilisation d'un principe actif selon l'invention sur la peau saine améliore significativement la migration cellulaire des kératinocytes et est capable d'induire la différenciation kératinocytaire. En améliorant ces deux processus biologiques via l'action des récepteurs sensoriels, le principe actif selon l'invention favorise la régénération épidermique de la peau.

Le principe actif selon l'invention est préférentiellement utilisé dans des compositions comprenant un milieu cosmétiquement acceptable. Il s'agit de compositions dans différentes formes galéniques, adaptées à une application par voie topique sur la peau.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect de crèmes, émulsions ou gels ou tous autres aspects des cosmétiques de soin peau.

Il peut s'agir de compositions comprenant au moins 0,5% d'un principe actif selon l'invention, préférentiellement entre 0,5 et 10%.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (*International Cosmetic Ingredient Dictionary and Handbook* publié par le *Personal Core Product Council*). Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées pour améliorer la qualité de la peau, l'embellir, en particulier pour un effet régénérant et/ou pour augmenter sa sensorialité. L'invention vise ainsi également un procédé non thérapeutique, à savoir un procédé cosmétique de traitement de la peau saine pour une augmentation de la sensorialité de la peau et/ou un effet régénérant de la peau, en particulier hydratant, lissant et/ou procurant un effet fraicheur, qui consiste en l'application topique sur la peau saine d'une composition comprenant un principe actif selon l'invention.

Préférentiellement la composition comprenant le principe actif cosmétique selon l'invention est appliquée au moins une fois par jour pendant au moins 15 jours.

Afin d'illustrer ces effets cosmétiques sur la qualité de la peau, en particulier sur les propriétés de la peau, les exemples suivants ainsi que des résultats d'essais sont présentés ci-dessous.

### EXEMPLES

### Exemple 1 : Principe actif selon l'invention

Le principe actif selon l'exemple 1 est obtenu par la mise en œuvre des étapes suivantes :
- solubilisation de 50g/L de farine d'albumen de coco dans un mélange eau/butylène glycol,
- Séparation des phases soluble et insoluble par filtration, et
- Filtrations successives.

Le principe actif obtenu se présente sous forme d'un liquide limpide de couleur jaune très clair. Il est constitué par :
- Une teneur en matières sèches de 28,9 g/l
- La présence de 1 ppm de butyl-O-alpha-D-glucopyranosyl-beta-D-glucopyranoside et de 4,3ppm de nonioside D,
- 17.3g/l, soit 60% de sucres en poids par rapport au poids de matière sèche, (déterminée par la méthode de DUBOIS), de degrés de polymérisation compris entre 2 et 7, constitués de saccharose,
- 2,5g/l, soit 9% de protéines en poids par rapport au poids de matière sèche, (déterminée par la méthode de KJELDHAL),
- 5g/l, soit 17% de cendres en poids par rapport au poids de matière sèche, (déterminée par la pesée des résidus issus de l'incinération des échantillons de l'hydrolysat à 550°C dans un four à moufle électrique).
- Moins de 1% de lipides

Ce principe actif selon l'invention est hydrosoluble.

### Exemple 2 : composition selon l'invention sous forme d'un soin de jour

Un exemple de composition selon l'invention sous forme d'un soin de jour est constitué par :

| | | | |
|---|---|---|---|
| A. | Eau | | qsp 100% |
| | Glycérine | | 3% |
| B. | Montanov 14 | (Seppic) | 2% |
| | DUB Zenoat | (Stéarinerie Dubois) | 2% |
| | Simulsol 165 | (Seppic) | 3% |
| | DUB MCT 5545 | (Stéarinerie Dubois) | 5% |
| C. | Sepimax Zen | (Seppic) | 0,8% |
| | Conservateur | | 1% |
| **C.** | **PRINCIPE ACTIF EXEMPLE 1** | **Invention** | **2,5%** |

La composition a un pH de 5,8. Elle se présente sous forme d'une émulsion blanche, brillante avec une texture souple. Son application est aisée e ne laisse pas de sensation de gras, avec une pénétration rapide et un fini sec. La composition peut être obtenue comme suit :
- Mélanger A. Chauffer au bain-marie à 80°C sous agitation magnétique.
- Mélanger B. Chauffer au bain-marie à 80°C sous agitation magnétique.
- A 80°C, ajouter B dans A, sous rotor stator à 1800 tr/min.
- A 50°C ajouter C sous rotor stator à 1500 tr/min.
- A 30°C, ajouter D.
- Laisser sous agitation jusqu'à complet refroidissement.

### Exemple 3 : composition selon l'invention sous forme d'un soin peaux matures

Un exemple de composition selon l'invention sous forme d'un soin peaux matures est constitué par :

| | | | |
|---|---|---|---|
| A. | Eau | | qsp 100% |
| | Conservateur | | 1% |
| | **PRINCIPE ACTIF EXEMPLE 1** | **Invention** | **2,5%** |
| | Sepiplus 400 | (Seppic) | 1% |
| | Glycérol | | 3% |
| | Keltrol CG | (CP Kelco) | 0,1% |
| | | | |
| B. | Lanol 99 | (Seppic) | 8% |
| | Lanol 1688 | (Seppic) | 5% |
| | Montanov 202 | (Seppic) | 2% |
| | Montanov 82 | (Seppic) | 1% |

La composition a un pH de 6. Elle se présente sous forme d'une émulsion blanche et brillante. Son étalement est aisé, elle fond sur la peau, et présente une pénétration rapide avec un fini doux. La composition peut être obtenue comme suit :
- Mélanger A. Chauffer au bain-marie à 80°C sous agitation magnétique.
- Mélanger B. Chauffer au bain-marie à 80°C sous agitation magnétique.
- A 80°C, ajouter B dans A sous rotor stator à 1800 tr/min.
- Laisser sous agitation jusqu'à complète homogénéisation.

### Exemple 4 : eau de coco pour essai comparatif avec le principe actif selon l'invention

L'eau de coco utilisée dans l'essai comparatif est disponible sous la référence « Eau de coco, Bio-équitable, Nature, La Maison du Coco, Origine Philippines ». Elle correspond à la partie liquide contenue à l'intérieur de l'albumen des noix de coco immatures.

Elle présente notamment les caractéristiques suivantes :
- Présence de nonioside D, sa teneur est évaluée comme étant inférieure à 2ppm.
- Absence de butyl-O-alpha-D-glucopyranosyl-beta-D-glucopyranoside

### Exemple 5 : huile de coco pour essai comparatif avec le principe actif selon l'invention

L'huile de coco utilisée dans l'essai comparatif est disponible sous la référence « Huile vierge de coco bio, Rapunzel, origine Sri Lanka ». Cette huile est obtenue après pression de l'albumen.

Elle présente notamment les caractéristiques suivantes :
- Absence de nonioside D,
- Absence de butyl-O-alpha-D-glucopyranosyl-beta-D-glucopyranoside

### ESSAIS ET RESULTATS

### I. Effet du principe actif selon l'invention sur les récepteurs olfactifs (test in vitro)

L'objectif de cette étude est d'évaluer la capacité d'un principe actif selon l'invention à restaurer l'expression des récepteurs olfactifs (OR : Olfactory receptors OR2A4/7, OR51B5 et OR2AT4 chez des sujets âgés, en comparaison avec un agoniste de ces récepteurs : le sandalore connu pour être capable d'activer le récepteur OR2AT4 et en présence ou non d'un antagoniste le phenirat^{®}.

L'étude a été réalisée:
- sur explants de peau humaine issus de donneurs jeunes (inférieur à 30 ans) ou de donneurs âgés (supérieur à 60 ans) par marquage immunohistologique ;
- sur kératinocytes humains issus de donneurs jeunes (âge inférieur à 25 ans) ou issus de donneurs âgés (âge supérieur à 60 ans) par PCR quantitative.

### Le protocole opératoire pour l'étude de la synthèse de OR2AT4 est décrit en suivant :

Pendant plusieurs jours, des explants issus de plasties humaines maintenus en survie dans du milieu de culture, sont traités en systémique avec :
- exemple 1 à 0,10% et 0,25% (V/V) ;
- exemple 1 à 0,25% + phenirat^{®} 50µM ;
- Sandalore 100µM ;
- Sandalore 100µM + phenirat^{®} 50µM.

Après plusieurs jours, les explants sont récupérés et congelés. Des coupes (4 µm) sont ensuite réalisées à l'aide d'un cryostat CM1850.

Un marquage immunohistologique est ensuite réalisé :
- Décongélation et réhydratation des coupes
- Saturation
- Anticorps primaire : anticorps anti-OR2AT4
- Anticorps secondaire : anticorps couplé Alexa Fluor^{®} 488

La visualisation est réalisée sur microscope couplé à un système d'analyse d'images.

La synthèse de OR2AT4 est proportionnelle à l'intensité de la fluorescence (couleur verte) présente sur les explants. Une analyse quantitative des images a été réalisée à l'aide d'un logiciel.

### Le protocole opératoire pour l'étude de l'expression des OR2A4/7 et OR51B5 est décrit en suivant :

A J0, les kératinocytes sont ensemencés dans du milieu de culture et incubés à 37°C.

Pendant plusieurs jours, les kératinocytes sont traités avec l'exemple 1 à 0,10% et 0,50% (V/V). Après quelques jours, les ARN ont été reverses-transcrits et les ADN complémentaires obtenus ont été analysés par la technique de PCR quantitative. L'expression des ARNm de OR2A4/7 et

OR51B5 a été étudiée, en parallèle d'ARNm témoins internes de référence pour la normalisation. L'analyse des Ct (quantification relative) est réalisée à l'aide d'un logiciel.

Les résultats obtenus concernant la synthèse de OR2AT4 sont présentés dans le Tableau 3 et ceux relatifs à la capacité de restaurer l'expression OR2A4/7 et OR51B dans le Tableau 4.

**Tableau 3. Effet de l'invention sur la synthèse de OR2AT4 dans des explants issus de donneurs jeunes et âgés.**

| | **Synthèse de OR2AT4 (UA)** | **Capacité à restaurer la synthèse de OR2AT4 (%)** |
|---|---|---|
| **Explants jeunes** | | |
| Témoin | 59 | |
| Exemple 1 0,25% | 60 | |

| **Explants âgés** | | |
|---|---|---|
| Témoin | 44 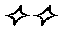 | - |
| Exemple 1 0,10% | 55**** | +73% |
| Exemple 1 0,25% | 58**** | +93% |
| Exemple 1 0,25% + phenirat^{®} 50µM | 46 | +13% |
| Sandalore 100µM | 57**** | +87% |
| Sandalore 100µM + phenirat^{®} 50µM | 47 | +20% |

| | | |
|---|---|---|
| 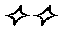 *résultat significatif selon le test t de Student* / *témoin explants jeunes (p < 0,01)* *** : résultats significatifs selon le test t de Student* / *témoin explants âgés (p < 0,01)* | | |

**Tableau 4. Effet de l'invention sur l'expression de OR2A4/7 et OR5185 par des kératinocytes humains issus de donneurs jeunes et âgés.**

| | **Expression de OR2A4/7 (%)** | **Capacité à restaurer l'expression de OR2A4/7 (%)** | **Expression de OR51B5 (%)** | **Capacité à restaurer l'expression de OR51B5 (%)** |
|---|---|---|---|---|
| **Kératinocytes jeunes** | | | | |
| Témoin | 100 | | 100 | |
| Exemple 1 0,50% | 102 | | 103 | |

| **Kératinocytes âgés** | | | | |
|---|---|---|---|---|
| Témoin | 39 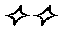 | - | 46 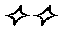 | - |
| Exemple 1 0,10% | 44 | +8% | 70 | +44% |
| Exemple 1 0,50% | 51** | +20% | 102** | +104% |

| | | | | |
|---|---|---|---|---|
| 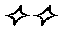 *: résultats significatifs selon le test t de Student* / *kératinocytes jeunes (p < 0,01)* ** *: résultats significatifs selon le test t de Student* / *kératinocytes âgés (p < 0,01)* | | | | |

Ces résultats démontrent qu'au cours du vieillissement, la présence des récepteurs olfactifs dans la peau est significativement diminuée. En effet, dans des kératinocytes âgés, l'expression de OR2A4/7 et OR51B5 est diminuée de respectivement 61% et 54%. De même, les explants humains âgés synthétisent moins de OR2AT4 (-25%).

Testé à 0,50% sur kératinocytes humains âgés, un principe actif selon l'invention augmente significativement l'expression de OR2A4/7 et de OR51B5 de respectivement 20% et 104%. Testé à 0,25% sur explants humains âgés, un principe actif selon l'invention restaure significativement la synthèse de OR2AT4 (+ 93%).

L'utilisation d'un principe actif selon l'invention permet ainsi le maintien de l'expression et la synthèse des récepteurs olfactifs épidermiques au cours du vieillissement.

### II. Effet du principe actif selon l'invention sur les récepteurs gustatifs (test in vitro)

L'objectif de cette étude est d'évaluer la capacité d'un principe actif selon l'invention à restaurer l'expression des récepteurs gustatifs TAS2R1 (Taste Receptor type 2 member 1) et TAS2R38 (Taste Receptor type 2 member 38) chez des sujets âgés, en comparaion à un agoniste de ces récepteurs, l'amarogentine qui intensifie l'expression des marqueurs impliqués dans la maturation et la différenciation terminale épidermique.

L'étude a été réalisée par immunohistofluorescence sur des explants de peau humaine issus de donneurs jeunes (inférieur à 30 ans) ou de donneurs âgés (supérieur à 60 ans)

Le protocole opératoire est décrit en suivant.

Pendant plusieurs jours, des explants issus de plasties humaines maintenus en survie dans du milieu de culture, sont traités en systémique avec :
- l'exemple 1 à 0,10% et 0,25% (V/V) ;
- l'amarogentine 100µM (agoniste des récepteurs gustatifs).

A J7, les explants sont récupérés et congelés. Des coupes (4 µm) sont ensuite réalisées à l'aide d'un cryostat.

Un marquage immunohistologique est ensuite réalisé :
- Décongélation et réhydratation des coupes
- Saturation
- Anticorps primaires :
   * Anticorps anti-TAS2R1
   * Anticorps anti-TAS2R38
- Anticorps secondaires :
   * Anticorps couplé Alexa Fluor^{®} 488

La visualisation est réalisée sur un microscope couplé à un système d'analyse d'images.

La synthèse de TAS2R1 et TAS2R38 est proportionnelle à l'intensité de la fluorescence (couleur verte) présente sur les explants. Une analyse quantitative des images a été réalisée à l'aide d'un logiciel.

Les résultats sont donnés dans le Tableau 5.

**Tableau 5. Effet de l'invention sur la synthèse de TAS2R1 et TAS2R38 sur des explants issus de donneurs jeunes et âgés.**

| | **Synthèse de TAS2R1 (UA)** | **Capacité à restaurer la synthèse de TAS2R1 (%)** | **Synthèse de TAS2R38 (UA)** | **Capacité à restaurer la synthèse de TAS2R38 (%)** |
|---|---|---|---|---|
| **Explants jeunes** | | | | |
| Témoin | 28 | | 29 | |
| Exemple 1 0,25% | 28 | | 28 | |

| **Explants âgés** | | | | |
|---|---|---|---|---|
| Témoin | 20 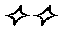 | - | 22 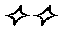 | - |
| Exemple 1 0,10% | 25 | +63% | 25**** | +43% |
| Exemple 1 0,25% | 27* | +88% | 28**** | +86% |
| Amarogentine 100µM | 26* | +75% | 28**** | +86% |

| | | | | |
|---|---|---|---|---|
| 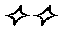 : *résultats significatifs selon le test t de Student* / *témoin explants jeunes (p < 0,01)* ** : résultats significatifs selon le test t de Student* / *témoin explants âgés (p < 0,05)* *** : résultats significatifs selon le test t de Student* / *témoin explants âgés (p < 0,01)* | | | | |

On constate qu'en comparaison aux explants jeunes, les explants humains âgés synthétisent significativement moins de récepteurs gustatifs TAS2R1 et TAS2R38 (-29% et -24% respectivement). Ceci traduit un effet du vieillissement sur la présence des récepteurs gustatifs dans la peau.

Testé à 0,25% sur explants humains âgés, un principe actif selon l'invention restaure significativement la synthèse de TAS2R1 de 88% et la synthèse de TAS2R38 de 86%. L'invention permet ainsi de maintenir une synthèse des récepteurs gustatifs épidermiques dans des cultures de kératinocytes âgés, équivalente à celle obtenue dans des cultures de kératinocytes jeunes.

### III. Effet du principe actif selon l'invention sur la régénération épidermique (tests in vitro)

### III.1 Effet d'un principe actif selon l'invention sur la migration kératinocytaire

L'objectif de cette étude est d'évaluer la capacité d'un principe actif selon l'invention à augmenter la migration cellulaire de kératinocytes âgés grâce à l'activation de récepteurs olfactifs, en comparaison au sandalore, molécule de référence, et en présence de phenirat qui quant à lui est un inhibiteur de ces récepteurs.

Cette étude a été réalisée par coloration histochimique sur des kératinocytes issus de donneurs jeunes (âge inférieur à 25 ans) ou issus de donneurs âgés (âge supérieur à 60 ans). Le protocole opératoire de l'essai est décrit en suivant.

A J0, les kératinocytes sont ensemencés dans une chambre de culture IBIDI^{®} et incubés à 37°C. Pendant plusieurs jours, les kératinocytes sont traités avec l'exemple 1 à 0,05% et 0,10% (V/V) ou le sandalore à 200µM, avec ou sans phenirat 100µM.

Après quelques jours, l'insert est retiré de la chambre de culture créant la blessure. Les cellules sont à nouveau traitées avec l'exemple 1 à 0,05% et 0,10% (V/V) ou le sandalore à 200µM, avec ou sans phenirat 100µM, pendant plusieurs jours.

Après quelques jours, les cellules sont colorées avec une solution de crystal violet à 0,5% (M/V).

La visualisation est réalisée avec une caméra sur microscope couplé à un système d'analyse d'images. Le ratio de surface occupée par les cellules est calculé de façon automatique grâce à un logiciel.

Les résultats sont résumés dans le Tableau 6.

**Tableau 6. Effet de l'invention sur la migration des kératinocytes issus de donneurs jeunes et âgés.**

| | **Ratio de surface occupée (UA)** | **Capacité à améliorer la migration cellulaire (%)** |
|---|---|---|
| **Kératinocytes jeunes** | | |
| Témoin | 0,739 | |
| Exemple 1 0,10% | 0,740 | |

| **Kératinocytes âgés** | | |
|---|---|---|
| Témoin | 0,351 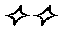 | |
| Exemple 1 0,05% | 0,431 | +23% |
| Exemple 1 0,10% | 0,491* | +40% |
| Exemple 1 0,10% + Phenirat 100µM | 0,353 | +1% |

| | | |
|---|---|---|
| Sandalore 200µM | 0,553** | +58% |
| Sandalore + Phenirat 100µM | 0,339 | +0% |

| | | |
|---|---|---|
| 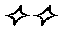 : *résultat significatif selon le test t de Student* / *témoin kératinocytes jeunes (p < 0,01)* **: résultat significatif selon le test t de Student* / *témoin kératinocytes âgés (p < 0,05)* ***: résultat significatif selon le test t de Student* / *témoin kératinocytes âgés (p < 0,01)* | | |

On constate qu'en comparaison aux kératinocytes humains jeunes, la capacité de migration des kératinocytes humains âgés est significativement diminuée (-53%).

Testé à 0,10% sur kératinocytes âgés, l'invention améliore significativement la migration cellulaire de +40%.

En présence de phenirat, molécule antagoniste des récepteurs olfactifs, un principe actif selon l'invention n'induit plus la migration des kératinocytes humains âgés.

Par conséquent, l'effet de l'invention sur la migration kératinocytaire résulte d'une activation des récepteurs olfactifs.

### III.2 Effet d'un principe actif selon l'invention sur la régénération de la fonction barrière

L'objectif de cette étude est d'évaluer la capacité d'un principe actif selon l'invention à restaurer la fonction barrière cutanée sur des peaux humaines altérées par agression mécanique.

Cette étude a été réalisée par immunohistologie sur des explants de peau humaine.

Le protocole opératoire est décrit en suivant.

Pendant plusieurs jours, la barrière cutanée d'explants de peau humaine a été agressée mécaniquement par application successive d'adhésifs

Des explants sont maintenus en survie dans du milieu de culture et sont traités tous les jours en systémique avec l'exemple 1 à 0,25% (V/V).

Après quelques jours de traitement, les explants sont récupérés fixés, déshydratés et inclus en paraffine. Des coupes (4 µm) sont ensuite réalisées à l'aide d'un microtome.

Une coloration Hématoxyline Eosine Safran (HES) est réalisée :
- Déparaffinage en xylène
- Hydratation
- Coloration hémalun de Masson ; éosine et safran
- Déshydratation
- Montage des lames

La visualisation est réalisée sur un microscope couplé à un système d'analyse d'images.

Les résultats de la coloration HES étant qualitatifs, 3 niveaux ont été définis sur l'aspect visuel du *stratum corneum :*

| | |
|---|---|
| *- stratum corneum* fin | + |
| *- stratum corneum* moyen | ++ |
| *- stratum corneum* épais | +++ |

Les résultats sont résumés dans le Tableau 7 et sur la Figure 1.

**Tableau 7. Effet de l'invention sur la régénération épidermique**

| | **Appréciation qualitative du stratum corneum** |
|---|---|
| **Explants non agressés** | |
| Témoin | +++ |

| **Explants agressés** | |
|---|---|
| Témoin | + |
| Exemple 10,25% | +++ |

On constate que l'agression mécanique des explants de peau humaine entraîne une altération des couches supérieures épidermiques et notamment du *stratum corneum.*

Testé à 0,25% sur explants agressés, un principe actif selon l'invention améliore la régénération épidermique.

### IV. Capacité du principe actif selon l'invention à améliorer la qualité de la barrière cutanée (test in vivo)

L'objectif de l'étude est d'évaluer, *in vivo,* l'effet d'un principe actif selon l'invention formulé à 2,5% en gel émulsionné fluide, sur la qualité de la barrière cutanée en comparaison au placebo, à la fois sur volontaires caucasiens et sur volontaires asiatiques.

La composition utilisée pour cet essai est la suivante :

**Tableau 8**

| | |
|---|---|
| Glycerol | 5,0% |
| Exemple 1 | 2,5% |
| PEG-7 glyceryl cocoate (DUB GC7, Dubois) | 1,2% |
| Conservateurs | 1,0% |
| Caprylic / capric triglycerides (DUB MCT 5545, Dubois) | 0,8% |
| Acrylate / C10-30 alkyl acrylate crosspolymer (Carbopol ULTREZ20, Noveon) | 0,3% |
| NaOH | qsp pH 4,5 |
| Eau | qsp 100 |

Le placebo a la même formulation sans le principe actif de l'exemple 1.

Les panels sont les suivants :

### • Panel caucasien :

Composé de 20 volontaires sains, de sexe féminin, d'âge compris entre 55 et 69 ans (âge moyen 63 ± 5 ans), ayant appliqué matin et soir l'invention et le placebo en hémi-visage pendant 14 jours.

### • Panel asiatique :

Composé de 33 volontaires sains d'origine chinoise, de sexe féminin, d'âge compris entre 56 et 69 ans (âge moyen 62 ± 3 ans), ayant appliqué matin et soir l'invention et le placebo en hémi-visage pendant 14 jours.

Des mesures de Perte insensible en eau (PIE) au niveau du visage ont été réalisées à l'aide d'un Tewamètre^{®} avant et après 14 jours de traitement biquotidien.

Un résumé des résultats correspondant à l'effet du principe actif selon l'invention, sur la PIE mesurée à l'aide d'un Tewamètre^{®} sur des volontaires caucasien et asiatique est présenté sur le tableau 9 ci-après.

**Tableau 9. Effet de l'invention formulé à 2,5% sur la fonction barrière après 14 jours d'application biquotidienne.**

| | PIE | | Δ/J0 | Δ/placebo |
|---|---|---|---|---|
| | J0 | J14 | | |
| Panel caucasien | | | | |
| placebo | 12.00 | 11.36 | -5.3% | |
| Principe actif 2.5% | 12.74 | 10.74 | -15.7% | 10.4% |

| Panel asiatique | | | | |
|---|---|---|---|---|
| placebo | 19.0 | 11.6 | -39.1% | |
| Principe actif 2.5% | 18.8 | 10.5 | -44.3% | 5.3% |

On constate que dans les conditions de cette étude, après 14 jours d'application biquotidienne, un principe actif selon l'invention formulé à 2,5% en gel émulsionné fluide diminue significativement la perte insensible en eau au niveau du visage :
- chez les volontaires du panel caucasien de 10,4% (p = 0,0392). Cet effet est observé chez 75% d'entre eux ;
- chez les sujets du panel asiatique de 5,3% (p = 0,0407). Cet effet est observé chez 63% d'entre eux.

### V. Capacité du principe actif à hydrater la peau (test in vivo)

### V .1. Etude de l'hydratation sur une journée

L'objectif de cette étude est d'évaluer, in vivo, l'effet hydratant sur une journée du principe actif selon l'invention formulé à 2,5% en émulsion, en comparaison au placebo.

Cette étude a été réalisée sur 14 volontaires caucasiens sains, de sexe féminin, âgés de 62 ± 4 ans, ayant appliqué le principe actif selon l'invention et le placebo en hémi-visage.

Les mesures du taux d'hydratation au niveau du visage ont été effectuées à l'aide d'un Cornéomètre^{®} avant application des traitements, puis 10 minutes, 1 heure, 3 heures et 6 heures après une application unique.

Un résumé des résultats correspondant à l'effet du principe actif selon l'invention est présenté sur le tableau 10 ci-après.

**Tableau 10. Effet de l'invention formulé à 2,5% sur l'hydratation cutanée sur une journée.**

| | Taux d'hydratation (UA) | | | | |
|---|---|---|---|---|---|
| | t0 | t10min | t1h | t3h | t6h |
| Placebo | 25,5 | 34,7 | 33,9 | 32,4 | 28,5 |
| Principe actif 2,5% | 27,6 | 45,9 | 45,2 | 42,3 | 40,5 |

On constate qu'en comparaison au placebo, dès l'application, le principe actif selon l'invention augmente significativement le taux d'hydratation (+29,6%, p = 0,0146). Cet effet persiste tout au long de la journée (+35,0%, p = 0,0167, 6 heures après l'application).

Ce résultat a été observé chez 79% des volontaires.

### V .2. Etude de l'hydratation à long terme

L'objectif de cette étude est d'évaluer, in vivo, l'effet hydratant à long terme du principe actif selon l'invention formulé à 2,5% en émulsion, en comparaison au placebo.

Cette étude a été réalisée sur 20 volontaires caucasiens sains, de sexe féminin, âgés de 65 ± 5 ans, présentant un teint terne et ayant appliqué matin et soir le principe actif selon l'invention et le placebo en hémi-visage.

Le taux d'hydratation a été mesuré à l'aide d'un Cornéomètre^{®} et le dosage du lactate a été effectué à partir de prélèvements réalisés au niveau des joues avant et après 28 jours de traitement biquotidien.

La concentration du lactate est un paramètre supplémentaire pour connaitre l'hydratation de la peau. En effet, il s'agit d'un Facteur Naturel d'Hydratation qui permet de fixer et d'assurer l'hydratation de la couche cornée et la souplesse de la peau. Plus la concentration du lactate est élevée, plus l'hydratation de la peau est importante.

Un résumé des résultats correspondant à l'effet du principe actif selon l'invention sur l'hydratation à long terme de la peau est présenté sur le tableau 11 ci-après.

**Tableau 11. Effet de l'invention formulé à 2,5% sur l'hydratation de la peau après 28 jours d'application biquotidienne.**

| | ***Variation* / *J0 (%)*** |
|---|---|
| Placebo | 5,8 |
| Principe actif 2,5% | 27,6 |
| Variation / Placebo (%) | 21,7 |

Un résumé des résultats correspondant à l'effet du principe actif selon l'invention sur la concentration en lactate est présenté sur le tableau 12 ci-après.

**Tableau 12. Effet de l'invention formulé à 2,5% sur la concentration en lactate après 28 jours d'application biquotidienne.**

| | ***Variation* / *J0 (%)*** |
|---|---|
| Placebo | 0,5 |
| Principe actif 2,5% | 50,3 |
| Variation / Placebo (%) | 49,8 |

On constate qu'après 28 jours d'application biquotidienne au niveau du visage et en comparaison au placebo, le principe actif selon l'invention formulé à 2,5% en émulsion augmente significativement :
- l'hydratation cutanée de 21,7% (p = 0,0008 ; effet validé chez 85% des volontaires) ;
- la concentration en lactate de 49,8% (p = 0,0225 ; résultat observé chez 70% des volontaires).

### VI. Effet du principe actif sur l'éclat du teint (test in vivo)

L'objectif de cette étude est d'évaluer, in vivo, l'effet du principe actif selon l'invention formulé à 2,5% en émulsion, sur l'éclat du teint de la peau, en comparaison au placebo.

Cette étude a été réalisée sur 20 volontaires caucasiens sains, de sexe féminin, âgés de 65 ± 5 ans, présentant un teint terne et ayant appliqué matin et soir le principe actif selon l'invention et le placebo en hémi-visage.

L'éclat du teint a été évalué par scorage clinique par deux experts entraînés, avant et après 28 jours d'application biquotidienne.

Un résumé des résultats correspondant à l'effet du principe actif selon l'invention sur les principaux paramètres caractéristiques de l'éclat du teint évalués par experts, est présenté dans le tableau 13.

**Tableau 13. Effet du principe actif selon l'invention sur l'éclat du teint après 28 jours d'application biquotidienne.**

| | Variation / Placebo (%) |
|---|---|
| Rayonnement | 10,6 |
| Couleur rose | 22,8 |
| Effet bonne mine | 23,4 |
| Couleur olive | -12,5 |
| Rides et ridules | -10,5 |
| Etat de fatigue des yeux | -8,3 |

Dans les conditions de cette étude, après 28 jours d'application biquotidienne et en comparaison au placebo, le principe actif selon l'invention formulé à 2,5% en émulsion améliore significativement l'éclat du teint :
- en augmentant le rayonnement de la peau (+10,6%, p = 0,0003) et la couleur rose représentative d'un teint frais (+22,8%, p = 0,0007), procurant ainsi un effet bonne mine (+23,4%, p = 0,0002) ;
- en diminuant la couleur olive (-12,5%, p = 0,0001), la présence de rides et ridules (-10,5%, p = 0,0002) et l'état de fatigue des yeux (-8,3%,
   p = 0,0016).

Ce résultat a été observé chez plus de 70% des volontaires pour chacun des items d'intérêt.

### VII. Capacité du principe actif à améliorer la qualité de la peau (test in vivo)

L'objectif de cette étude est d'évaluer, in vivo, les sensations observées lors de l'utilisation du principe actif selon l'invention formulé à 2,5% en émulsion, en comparaison à son placebo. Cette étude a été réalisée à l'aide d'un questionnaire d'auto-évaluation sur 20 volontaires caucasiens sains, de sexe féminin, âgés de 60 à 70 ans, présentant un teint terne et ayant appliqué matin et soir le principe actif selon l'invention et le placebo en hémi-visage. L'évaluation a été réalisée après 28 jours d'application biquotidienne.

Les résultats des questions fermées sont présentées dans le tableau 14 :

**Tableau 14**

| | Placebo | Principe actif 2,5% |
|---|---|---|
| Peau plus hydratée (*) | 60 | 80 |
| Peau mieux nourrie (ns) | 65 | 80 |
| Peau plus lisse (*) | 75 | 95 |
| Teint plus éclatant (ns) | 65 | 80 |
| Vitalité retrouvée (*) | 40 | 60 |
| Visage rajeuni (*) | 30 | 55 |

| | | |
|---|---|---|
| *^{ns}: différences non significatives* ** : différences significatives* / *placebo (p < 0,05)* | | |

Dans les conditions de cette étude, après 28 jours d'application biquotidienne et en comparaison à un placebo, le principe actif selon l'invention formulé à 2,5% est perçu par les volontaires comme globalement plus efficace.

Leur peau leur paraît plus lisse et plus hydratée. Elles estiment que le principe actif selon l'invention permet à la peau de retrouver sa vitalité et le visage paraît ainsi rajeuni.

### VIII. Capacité du principe actif à améliorer la qualité de la peau (test in vivo)

L'objectif de cette étude est de comparer l'efficacité d'un soin contenant le principe actif selon l'invention formulé à 2.5% à son placebo.

Cette étude a été réalisée à l'aide de questionnaires d'auto-évaluation sur 150 femmes vivant en France, d'âge compris entre 55 et 70 ans, ayant tous types de peaux du visage sans quota de sensibilité.

Les volontaires ont été réparties en 2 groupes :
- Groupe Principe actif : 75 femmes d'âge moyen 61 +/- 4 ans ;
- Groupe placebo : 75 femmes d'âge moyen 61 +/- 4 ans.

Les évaluations ont été réalisées après une première journée d'application ainsi qu'après 14 jours d'application biquotidienne.

Après une première journée d'application, les volontaires ont jugées les deux produits. Elles considèrent favorables les items suivants :

**Tableau 15**

| | % de volontaires | |
|---|---|---|
| | Groupe placebo | Groupe Principe actif |
| Peau confortable(*) | 81 | 91 |
| Peau douce(**) | 77 | 93 |
| Peau hydratée(*) | 76 | 85 |
| Peau plus lisse(*) | 69 | 76 |
| Teint éclatant(**) | 44 | 61 |

| | | |
|---|---|---|
| **: résultat significatif selon le test de Wilcoxon-Mann-Whitney (p < 0,05)* ***: résultat significatif selon le test de Wilcoxon-Mann-Whitney (p < 0,01)* | | |

Les réponses à la question « Pendant combien de temps avez-vous ressenti un effet hydratant ? » sont les suivants :

**Tableau 16**

| | % de volontaires | |
|---|---|---|
| | Groupe placebo | Groupe principe actif |
| Moins d'1h | 52 | 29 |
| Entre 1h et 3h | 28 | 28 |
| Entre 3h et 6h | 17 | 19 |
| Entre 6h et 9h | 1 | 20 |
| Plus de 9h | 1 | 4 |

A la fin de la première journée d'application, les femmes ayant testé le principe actif selon l'invention formulé à 2,5% ont majoritairement trouvé leur peau plus douce, plus hydratée et plus confortable que le groupe de femmes ayant appliqué la formule placebo (différences significatives entre les deux groupes).

Près de la moitié d'entre elles ont évalué que la sensation d'hydratation avait duré plus de 3 heures contre seulement 19% des femmes ayant testé la formule placebo (différence significative).

Enfin, elles ont également trouvé leur peau plus lisse et plus éclatante (différences significatives).

Après 14 jours d'application, les volontaires ont jugé les deux produits. Elles considèrent favorables les items suivants :

**Tableau 17**

| | % de volontaires | |
|---|---|---|
| | Groupe placebo | Groupe Principe actif |
| Peau douce(ns) | 73 | 87 |
| Peau plus lisse(*) | 73 | 83 |
| Peau hydratée(**) | 60 | 77 |
| Teint éclatant(*) | 60 | 68 |
| Peau confortable(*) | 63 | 79 |
| Vitalité retrouvée (*) | 53 | 65 |

| | | |
|---|---|---|
| *ns : résultat non significatif* **: résultat significatif selon le test de Wilcoxon-Mann-Whitney (p < 0,05)* ***: résultat significatif selon le test de Wilcoxon-Mann-Whitney (p < 0,01)* | | |

Après 14 jours d'utilisation biquotidienne, les femmes ayant utilisé le principe actif selon l'invention formulé à 2,5% ont attribué de meilleurs résultats pour chaque item versus le groupe de femmes ayant testé la formule placebo.

Elles notent que leur peau est plus confortable et hydratée (différence significative par rapport au placebo).

De plus, il y a significativement plus de femmes dans le groupe principe actif à avoir perçu leur visage plus éclatant et à noter une vitalité de la peau retrouvée.

### VI. Cartographie sensorielle

Le ressenti et les émotions véhiculées par la texture d'un produit cosmétique sont devenus un critère essentiel dans le choix des consommateurs. En effet, si les consommateurs choisissent généralement un produit pour sa fonctionnalité ou sa promesse d'efficacité, ils vont particulièrement être séduits par la sensorialité du produit. Une sensorialité élevée est une source de bien-être, de plaisir et ceci est directement lié aux propriétés de texture du produit. Une des méthodes utilisées pour étudier les caractéristiques sensorielles d'un produit est le profil sensoriel descriptif quantitatif. Un profil sensoriel est la description des propriétés sensorielles d'un échantillon, où le panel d'experts évalue les échantillons au moyen d'une liste commune de descripteurs dont ils notent l'intensité (Norme NF EN ISO 13299 :2016-06). Ainsi, l'objectif de cette étude a été de caractériser le principe actif selon l'invention et d'étudier son influence et son apport sensoriel en formule.

Cette étude a été réalisée par un panel d'experts composé de 12 personnes d'âge compris entre 26 et 46 ans (âge moyen 36 ± 6 ans).

Les résultats obtenus sont présentés ci-après.

Le profil sensoriel du principe actif de l'exemple 1 incorporé dans l'eau à différentes concentrations, est présenté dans le Tableau 13.

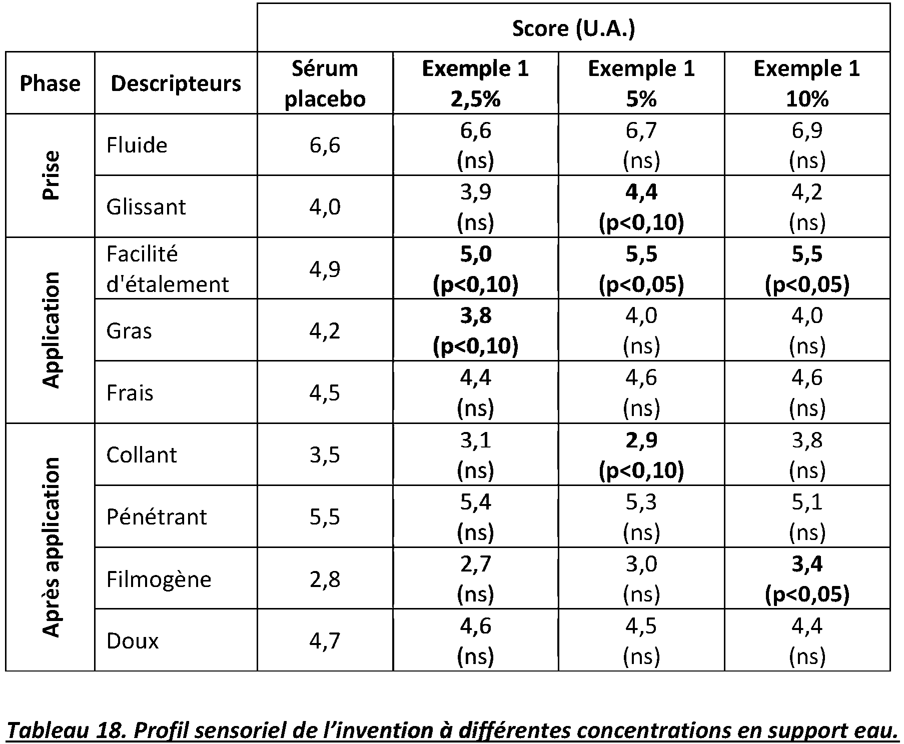

La signature sensorielle d'un actif selon l'invention montre un actif qui joue sur les propriétés rhéologiques en favorisant l'étalement (effet observé significativement dès 2,5 %).

De plus, le descripteur filmogène est également mis en évidence à 10%. Enfin, le principe actif selon l'invention ne confère pas d'effet collant ou gras.

Le profil sensoriel du principe actif de l'exemple 1, formulé en sérum, est présenté dans le Tableau 19.

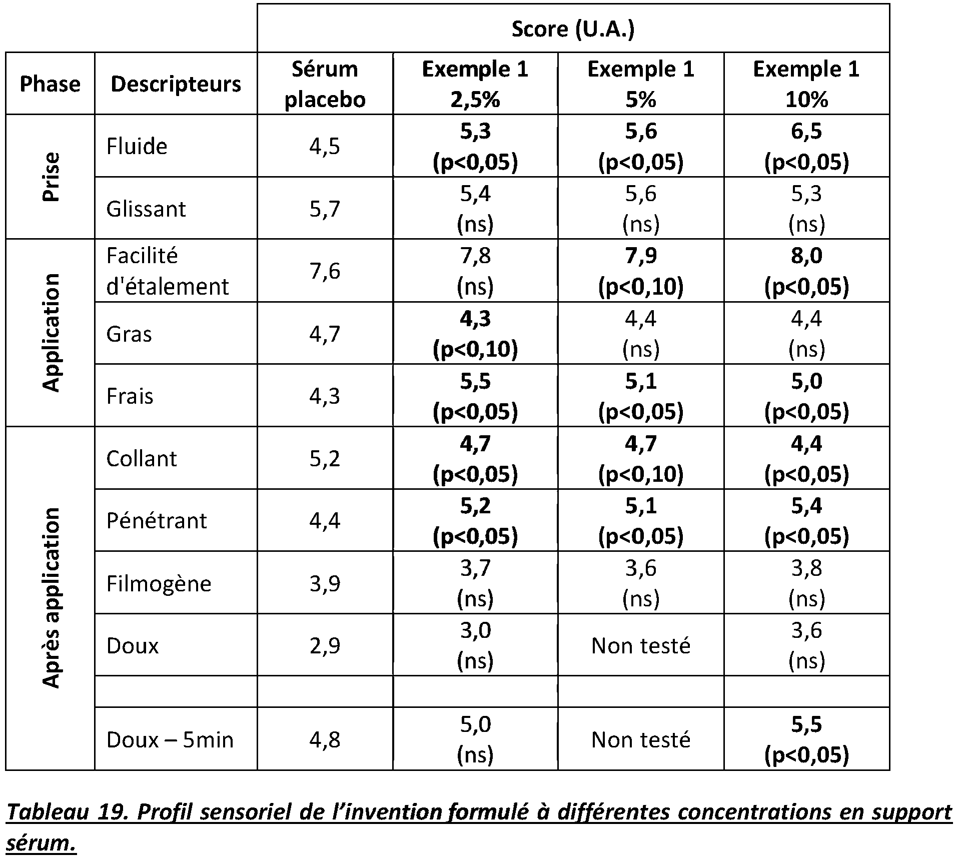

On constate que formulé dans un sérum, le principe actif selon l'invention dès 2,5%:
- confère plus de fluidité à la formule permettant d'offrir une consistance adaptée aux conditionnements sous forme de spray ;
- favorise l'étalement ;
- apporte une sensation de fraîcheur associée aux perceptions de bien-être ou encore de pureté ;
- favorise la pénétration du sérum ;
- réduit la perception de collant apporté par la formule.

La formulation en sérum permet l'expression de la signature sensorielle d'un actif selon l'invention De plus, le principe actif selon l'invention formulé à 2,5% et 10% augmente la perception de douceur transmise par la formule placebo (différence significative pour l'actif formulé à 10%).

Le profil sensoriel du principe actif selon l'invention, formulé en crème est présenté dans le Tableau 20.

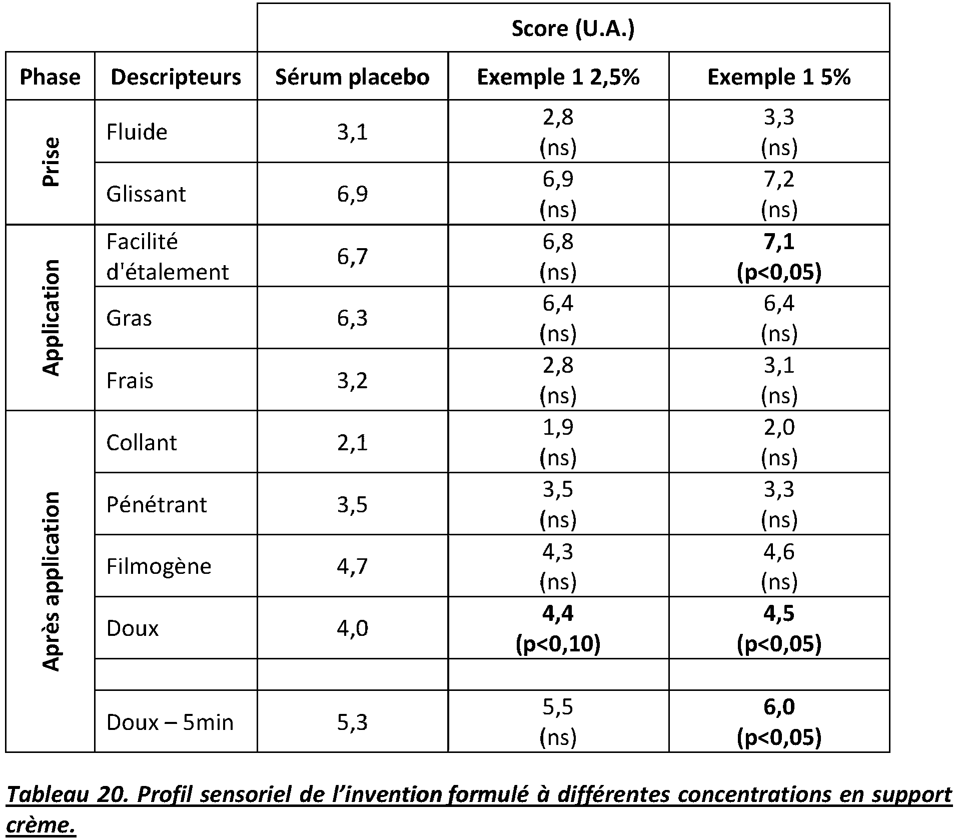

Formulé dans une crème à 2,5% ou 5%, le principe actif selon l'invention :
- ne fluidifie pas la formule ;
- favorise l'étalement de la crème à la surface de la peau de façon significative à 5% ;
- n'apporte pas de sensation collante.

De plus, le principe actif selon l'invention formulé à 5% augmente significativement la perception de douceur apportée par la crème placebo.

Le principe actif selon l'invention, en favorisant l'étalement et en limitant l'effet collant, permet à la douceur, composante multidimensionnelle, de s'exprimer. Le geste d'effleurement est ainsi facilité.

Incorporé en sérum ou en crème, le principe actif selon l'invention est adapté à la formulation de produits légers et non collants et permet de conférer de la douceur et de la fraîcheur aux formules, qualités cosmétiques recherchées par les consommateurs.

### VII. Essais comparatifs : comparaison de l'efficacité d'un principe actif selon l'invention avec l'eau de coco et l'huile de coco

### VII.1 Essai comparatif sur l'expression du récepteur olfactif OR2A4/7

L'objectif de cette étude est de comparer la capacité d'un principe actif selon l'invention, de l'eau de coco et d'huile de coco à restaurer l'expression des récepteurs olfactifs OR2A4/7 chez des sujets âgés.

L'étude a été réalisée sur kératinocytes humains issus de donneurs jeunes (âge inférieur à 25 ans) ou issus de donneurs âgés (âge supérieur à 60 ans) par PCR quantitative.

Le protocole opératoire est le suivant.

A J0, les kératinocytes sont ensemencés dans du milieu de culture et incubés à 37°C dans une atmosphère contenant 5% de CO₂.

A J3, J4, J5 les kératinocytes sont traités pendant 3 jours avec l'invention (exemple 1) ou de l'eau de coco (exemple 4) ou de l'huile de coco (exemple 5) à 0,50% (V/V).

A J6, T72h, les ARN ont été reverses-transcrits et les ADN complémentaires obtenus ont été analysés par la technique de PCR quantitative.

L'expression des ARNm de OR2A4/7 a été étudiée. En parallèle, les ARNm de la bêta-Glucuronidase (GUSB) et de la Ribosomal Protein S18 (RPS18) ont été analysés comme témoins internes de référence pour la normalisation.

La fluorescence (SYBR Green) est mesurée en continu à l'aide d'un thermocycleur. L'analyse des Ct (quantification relative) est réalisée à l'aide d'un logiciel.

Les résultats sont donnés dans le Tableau 21.

**Tableau 21. Effet de l'invention, de l'EAU DE COCO et de l'HUILE DE COCO sur l'expression de OR2A4/7 par des kératinocytes humains issus de donneurs âgés.**

| | **Expression de OR2A4/7 (%)** | **Capacité à restaurer l'expression de OR2A4/7 (%)** |
|---|---|---|
| **Kératinocytes jeunes** | | |
| Témoin | 100 | |

| **Kératinocytes âgés** | | |
|---|---|---|
| Témoin | 39 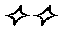 | - |
| Exemple 10,50% | 51** | +20% |
| EAU DE COCO (exemple 4) 0,50% | 37 | 0% |
| HUILE DE COCO (exemple 5) 0,50% | 39 | 0% |

| | | |
|---|---|---|
| 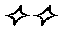 *: résultats significatifs selon le test t de Student* / *témoin kératinocytes jeunes (p < 0,01)* ***: résultats significatifs selon le test t de Student* /*témoin kératinocytes âgés (p < 0,01)* | | |

On constate que testé à 0,50% sur kératinocytes humains âgés, l'eau de coco et l'huile de coco ne restaurent pas l'expression de OR2A4/7 contrairement à l'invention.

### VII.2 Essai comparatif sur la migration kératinocytaire

L'objectif de cette étude est de comparer la capacité d'un principe actif selon l'invention, de l'eau de coco et d'huile de coco à augmenter la migration cellulaire de kératinocytes âgés grâce à l'activation de récepteurs olfactifs.

Cette étude a été réalisée par coloration histochimique sur des kératinocytes issus de donneurs jeunes (âge inférieur à 25 ans) ou issus de donneurs âgés (âge supérieur à 60 ans). Le protocole opératoire est le suivant.

A J0, les kératinocytes sont ensemencés dans une chambre de culture et incubés à 37°C.

A J3, les kératinocytes sont traités avec l'invention (exemple 1) ou de l'eau de coco (exemple 4) ou de l'huile de coco (exemple 5) à 0,10% (V/V).

A J4, formation de la zone de blessure,

L'insert est retiré de la chambre de culture créant la blessure. Les cellules sont à nouveau traitées avec l'invention (exemple 1) ou de l'eau de coco (exemple 5) ou de l'huile de coco (exemple 6) à 0,10% (V/V).

A J5, les kératinocytes sont traités avec l'invention (exemple 1) ou de l'eau de coco (exemple 4) ou de l'huile de coco (exemple 5) à 0,10% (V/V).

A J7, les cellules sont colorées avec une solution de crystal violet à 0,50% (M/V).

La visualisation est réalisée avec une caméra sur microscope couplé à un système d'analyse d'images.

Le ratio de surface occupée par les cellules est calculé de façon automatique grâce à un logiciel.

Les résultats sont résumés dans le Tableau 22.

**Tableau 22. Effet de l'invention, de l'eau de coco et de l'huile de coco sur la migration des kératinocytes issus de donneurs âgés.**

| | **Ratio surface occupée (UA)** | **Capacité à améliorer la migration cellulaire (%)** |
|---|---|---|
| **Kératinocytes jeunes** | | |
| Témoin | 0,739 | |

| **Kératinocytes âgés** | | |
|---|---|---|
| Témoin | 0,351 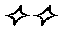 | / |
| Exemple 10,10% | 0,491* | +40% |
| EAU DE COCO (exemple 4) 0,50% | 0,270 | 0% |
| HUILE DE COCO (exemple 5) 0,50% | 0,228 | 0% |

| | | |
|---|---|---|
| 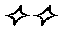 *: résultat significatif selon le test t de Student* / *témoin kératinocytes jeunes (p < 0,01)* **: résultat significatif selon le test t de Student* / *témoin kératinocytes âgés (p < 0,05)* | | |

La capacité de migration des kératinocytes humains âgés est significativement diminuée (-53%) comparé aux kératinocytes humains jeunes.

Testé à 0,10% sur kératinocytes âgés, l'eau de coco et l'huile de coco ne permettent pas d'améliorer la migration cellulaire contrairement à l'invention.

### VIII. Essai pour déterminer la fraction active du principe actif selon l'invention

Afin de déterminer la fraction active du principe actif selon l'invention, un fractionnement du principe actif a été réalisé.

Une fraction A est réalisée afin de purifier par adsorption successives des composés cationiques puis anioniques sur des résines ioniques. La fraction A est purifiée en sucres, elle contient 100% des sucres du principe actif selon l'invention et ne contient pas de protéines. L'efficacité de la fraction A est comparée à celle du principe actif selon l'invention sur l'expression du récepteur olfactif OR2A4/7 sur kératinocytes âgées, selon le protocole décrit au point I de la partie résultats d'essais.

Les résultats sont les suivants :

**Tableau 23**

| | Expression de OR2A4/7 | Capacité à restaurer l'expression de OR2A4/7 (%) |
|---|---|---|
| Kératinocytes jeunes | | |
| Témoin | 100 | |

| Kératinocytes âgés | | |
|---|---|---|
| Témoin | 37 | |
| Principe actif selon l'invention 0.5% (exemple 1) | 48 | -18% |
| Fraction A 0.5% | 47 | +17% |

La fraction A purifiée en sucres, possède une efficacité comparable à celle du principe actif selon l'invention, suggérant que l'activité de l'actif est conférée par les espèces glucidiques. Une étude métabolomique du principe actif et de la fraction A permet d'identifier les marqueurs responsables de l'efficacité.

L'identité de ces marqueurs a été établie par comparaison à des molécules identifiées dans la bibliographie de Cocos nucifera et aux molécules de bases de données.

Sur la base de l'analyse métabolomique, des disaccharides lipidiques présents dans les lots conformes en efficacité ont été identifiés, et en particulier deux marqueurs de type glycolipides : le nonioside D et le butyl-4-O-alpha-D-glucopyranosyl-beta-D-glucopyranoside.

## Revendications

1. Principe actif hydrosoluble cosmétique **caractérisé en ce que** ledit principe actif est un extrait hydrosoluble obtenu à partir de l'albumen du fruit de *Cocos nucifera,* ledit principe actif comprenant au moins deux disaccharides lipidiques, ledit principe étant susceptible d'être obtenu par un procédé comprenant les étapes suivantes :
a. solubilisation d'albumen de fruit de *Cocos nucifera* dans un mélange hydroglycolique,
b. séparation des phases soluble et insoluble par décantation,
c. filtrations successives.

2. Principe actif cosmétique selon la revendication précédente, caractérisé en que les disaccharides lipidiques présents sont au moins le butyl-O-alpha-D-glucopyranosyl-beta-D-glucopyranoside et le nonioside D.

3. Principe actif cosmétique selon la revendication précédente, caractérisé en que le principe actif comprend au moins 3 ppm de nonioside D et/ou au moins 0.3ppm de butyl-O-alpha-D-glucopyranosyl-beta-D-glucopyranoside

4. Principe actif cosmétique selon l'une des précédentes revendications, **caractérisé en ce qu'**il comprend également au moins un autre glycolipide en plus des disaccharides lipidiques.

5. Principe actif cosmétique selon l'une des précédentes revendications, **caractérisé en ce que** le principe actif est obtenu par extraction hydroglycolique de l'albumen du fruit de *Cocos nucifera.*

6. Principe actif cosmétique selon l'une des précédentes revendications, **caractérisé en ce que** le principe actif comprend au moins 40% de sucres en poids par rapport au poids de matières sèche du principe actif.

7. Principe actif selon la précédente revendication, **caractérisé en ce que** les sucres ont des degrés de polymérisation compris entre 2 et 10.

8. Principe actif cosmétique selon l'une des précédentes revendications, **caractérisé en ce que** le principe actif présente au moins l'une des caractéristiques suivantes :
- il comprend entre 10 et 50% de cendres minérales en poids par rapport au poids de matières sèches du principe actif
- il comprend moins de 10% de protéines en poids par rapport au poids de matières sèches du principe actif,
- il comprend moins de 1% de lipides en poids par rapport au poids de matières sèches du principe actif.

9. Principe actif selon l'une des précédentes revendications, **caractérisé en ce qu'**il se présente sous forme liquide.

10. Utilisation cosmétique d'un principe actif cosmétique issu de l'albumen du fruit de *Cocos nucifera* selon l'une des revendications 1 à 9 ou d'une composition en contenant, en application topique sur la peau pour embellir la peau.

11. Utilisation cosmétique d'un principe actif issu de l'albumen du fruit de *Cocos nucifera* selon l'une des revendications 1 à 9 ou d'une composition en contenant, en application topique sur la peau pour un effet régénérant de la peau.

12. Utilisation cosmétique d'un principe actif issu de l'albumen du fruit de *Cocos nucifera* selon l'une des revendications 1 à 9 ou d'une composition en contenant, en application topique sur la peau pour améliorer la qualité de la fonction barrière cutanée.

13. Utilisation cosmétique d'un principe actif cosmétique issu de l'albumen du fruit de *Cocos nucifera* selon l'une des revendications 1 à 9 ou d'une composition en contenant, en application topique sur la peau pour lisser et/ou hydrater la peau.

14. Utilisation cosmétique d'un principe actif issu de l'albumen du fruit de *Cocos nucifera* selon l'une des revendications 1 à 9 ou d'une composition en contenant, en application topique sur la peau pour agir sur les récepteurs olfactifs OR2A4 et/ou OR51B5 présentant dans les kératinocytes.

15. Utilisation cosmétique d'un principe actif issu de l'albumen du fruit de *Cocos nucifera* selon l'une des revendications 1 à 9 ou d'une composition en contenant, en application topique sur la peau pour agir sur les récepteurs gustatifs TAS2R1 présentant dans les kératinocytes.

16. Utilisation cosmétique non thérapeutique d'un principe actif cosmétique issu de l'albumen du fruit de *Cocos nucifera* selon l'une des revendications 1 à 9 ou d'une composition en contenant, en application topique sur une peau saine pour améliorer la migration kératinocytaire.

17. Utilisation cosmétique non thérapeutique d'un principe actif cosmétique issu de l'albumen du fruit de *Cocos nucifera* selon l'une des revendications 1 à 9 ou d'une composition en contenant, en application topique sur la peau pour augmenter la sensorialité de la peau.

18. Composition cosmétique comprenant au moins 0,5% en poids d'un principe actif selon l'une des revendications 1 à 9.

19. Composition cosmétique selon la revendication 18, **caractérisée en ce qu'**elle se présente sous forme de gel, émulsion ou crème.

20. Utilisation cosmétique d'une composition selon l'une des revendications 18 ou 19 dans un procédé cosmétique de traitement d'une peau pour un effet régénérant de la peau, **caractérisé en ce qu'**il consiste en l'application topique sur la peau de ladite composition.

21. Procédé cosmétique de traitement d'une peau saine pour un effet hydratant et/ou lissant et/ou pour augmenter la sensorialité de la peau, qui consiste en l'application topique sur la peau d'une composition selon l'une des revendications 18 ou 19.

## Patentansprüche

1. Kosmetischer wasserlöslicher Wirkstoff, **dadurch gekennzeichnet, dass** der Wirkstoff ein wasserlöslicher Extrakt ist, der aus dem Nährgewebe der Frucht von *Cocos nucifera,* erhalten wird, wobei der Wirkstoff mindestens zwei Lipiddisaccharide umfasst, wobei der Wirkstoff durch ein Verfahren erhalten werden kann, das die folgenden Schritte umfasst:
a. Solubilisieren von Nährgewebe der Frucht von *Cocos nucifera* in einem Hydroglykolgemisch,
b. Trennen der löslichen und unlöslichen Phasen durch Dekantieren,
c. aufeinanderfolgendes Filtrieren.

2. Kosmetischer Wirkstoff nach dem vorstehenden Anspruch,
**dadurch gekennzeichnet, dass** die vorhandenen Lipiddisaccharide mindestens Butyl-O-alpha-D-glucopyranosyl-beta-D-glucopyranosid und Noniosid D sind.

3. Kosmetischer Wirkstoff nach dem vorstehenden Anspruch,
**dadurch gekennzeichnet, dass** der Wirkstoff mindestens 3 ppm Noniosid D und/oder mindestens 0,3 ppm Butyl-O-alpha-D-glucopyranosyl-beta-D-glucopyranosid umfasst.

4. Kosmetischer Wirkstoff nach einem der vorstehenden Ansprüche, **dadurch gekennzeichnet, dass** er neben den Lipiddisacchariden auch mindestens ein weiteres Glykolipid umfasst.

5. Kosmetischer Wirkstoff nach einem der vorstehenden Ansprüche, **dadurch gekennzeichnet, dass** der Wirkstoff durch hydroglykolische Extraktion des Nährgewebes der Frucht von *Cocos nucifera* erhalten wird.

6. Kosmetischer Wirkstoff nach einem der vorstehenden Ansprüche, **dadurch gekennzeichnet, dass** der Wirkstoff bezogen auf das Trockengewicht des Wirkstoffs mindestens 40 Gew.-% Zucker umfasst.

7. Wirkstoff nach dem vorstehenden Anspruch,
**dadurch gekennzeichnet, dass** die Zucker Polymerisationsgrade zwischen 2 und 10 aufweisen.

8. Kosmetischer Wirkstoff nach einem der vorstehenden Ansprüche, **dadurch gekennzeichnet, dass** der Wirkstoff mindestens eine der folgenden Eigenschaften aufweist:
- er umfasst bezogen auf das Trockengewicht des Wirkstoffs zwischen 10 und 50 Gew.-% Mineralasche,
- er umfasst bezogen auf das Trockengewicht des Wirkstoffs weniger als 10 Gew.-% Proteine,
- er umfasst bezogen auf das Trockengewicht des Wirkstoffs weniger als 1 Gew.-% Lipide.

9. Wirkstoff nach einem der vorstehenden Ansprüche,
**dadurch gekennzeichnet, dass** er in flüssiger Form vorliegt.

10. Kosmetische Nutzung eines kosmetischen Wirkstoffs aus dem Nährgewebe der Frucht von Cocos *nucifera* nach einem der Ansprüche 1 bis 9 oder einer diesen enthaltenden Zusammensetzung zum topischen Auftragen auf die Haut zur Verschönerung der Haut.

11. Kosmetische Nutzung eines Wirkstoffs aus dem Nährgewebe der Frucht von Cocos *nucifera* nach einem der Ansprüche 1 bis 9 oder einer diesen enthaltenden Zusammensetzung zum topischen Auftragen auf die Haut für eine hautregenerierende Wirkung.

12. Kosmetische Nutzung eines Wirkstoffs aus dem Nährgewebe der Frucht von Cocos *nucifera* nach einem der Ansprüche 1 bis 9 oder einer diesen enthaltenden Zusammensetzung zum topischen Auftragen auf die Haut, um die Qualität der Hautbarrierefunktion zu verbessern.

13. Kosmetische Nutzung eines kosmetischen Wirkstoffs aus dem Nährgewebe der Frucht von Cocos *nucifera* nach einem der Ansprüche 1 bis 9 oder einer diesen enthaltenden Zusammensetzung zum topischen Auftragen auf die Haut, um die Haut zu glätten und/oder mit Feuchtigkeit zu versorgen.

14. Kosmetische Nutzung eines Wirkstoffs aus dem Nährgewebe der Frucht von *Cocos nucifera* nach einem der Ansprüche 1 bis 9 oder einer diesen enthaltenden Zusammensetzung zum topischen Auftragen auf die Haut, um auf die in den Keratinozyten vorhandenen Geruchsrezeptoren OR2A4 und/oder OR51B5 einzuwirken.

15. Kosmetische Nutzung eines Wirkstoffs aus dem Nährgewebe der Frucht von *Cocos nucifera* nach einem der Ansprüche 1 bis 9 oder einer diesen enthaltenden Zusammensetzung zum topischen Auftragen auf die Haut, um auf die in den Keratinozyten vorhandenen Geschmacksrezeptoren TAS2R1 einzuwirken.

16. Nichttherapeutische kosmetische Nutzung eines kosmetischen Wirkstoffs aus dem Nährgewebe der Frucht von *Cocos nucifera* nach einem der Ansprüche 1 bis 9 oder einer diesen enthaltenden Zusammensetzung zum topischen Auftragen auf die Haut zur Verbesserung der Keratinozytenmigration.

17. Nichttherapeutische kosmetische Nutzung eines kosmetischen Wirkstoffs aus dem Nährgewebe der Frucht von *Cocos nucifera* nach einem der Ansprüche 1 bis 9 oder einer diesen enthaltenden Zusammensetzung zum topischen Auftragen auf die Haut zur Verbesserung der sensorischen Eigenschaften der Haut.

18. Kosmetische Zusammensetzung, umfassend mindestens 0,5 Gew.-% eines Wirkstoffs nach einem der Ansprüche 1 bis 9.

19. Kosmetische Zusammensetzung nach dem Anspruch 18,
**dadurch gekennzeichnet, dass** sie in Form eines Gels, einer Emulsion oder einer Creme vorliegt.

20. Kosmetische Nutzung einer Zusammensetzung nach einem der Ansprüche 18 oder 19 in einem kosmetischen Verfahren zur Behandlung einer Haut für eine die Haut regenerierende Wirkung, **dadurch gekennzeichnet, dass** sie aus dem topischen Auftragen der Zusammensetzung auf die Haut besteht.

21. Kosmetisches Verfahren zur Behandlung einer gesunden Haut für eine feuchtigkeitsspendende und/oder glättende Wirkung und/oder zur Erhöhung der sensorischen Eigenschaften der Haut, das im topischen Auftragen einer Zusammensetzung nach einem der Ansprüche 18 oder 19 auf die Haut besteht.

## Claims

1. A water-soluble cosmetic active ingredient, **characterized in that** said active ingredient is a water-soluble extract obtained from the endosperm of the fruit of Cocos *nucifera,* said active ingredient comprising at least two lipid disaccharides, said ingredient being obtainable by a method comprising the following steps:
a. solubilizing endosperm of the fruit of Cocos *nucifera* in a hydroglycolic mixture,
b. separating the soluble and insoluble phases by decantation,
c. applying successive filtrations.

2. The cosmetic active ingredient according to the preceding claim, **characterized in that** the lipid disaccharides present are at least butyl-O-alpha-D-glucopyranosyl-beta-D-glucopyranoside and nonioside D.

3. The cosmetic active ingredient according to the preceding claim, **characterized in that** the active ingredient comprises at least 3 ppm of nonioside D and/or at least 0.3 ppm of butyl-O-alpha-D-glucopyranosyl-beta-D-glucopyranoside.

4. The cosmetic active ingredient according to one of the preceding claims, **characterized in that** it also comprises at least one other glycolipid in addition to the lipid disaccharides.

5. The cosmetic active ingredient according to one of the preceding claims, **characterized in that** the active ingredient is obtained by hydroglycolic extraction of the endosperm of the fruit of *Cocos nucifera.*

6. The cosmetic active ingredient according to one of the preceding claims, **characterized in that** the active ingredient comprises at least 40% by weight of sugars relative to the dry weight of the active ingredient.

7. The active ingredient according to the preceding claim, **characterized in that** the sugars have degrees of polymerization ranging from 2 to 10.

8. The cosmetic active ingredient according to one of the preceding claims, **characterized in that** the active ingredient has at least one of the following characteristics:
- it comprises between 10% and 50% by weight of mineral ash relative to the dry weight of the active ingredient
- it comprises less than 10% by weight of proteins relative to the dry weight of the active ingredient,
- it comprises less than 1% by weight of lipids relative to the dry weight of the active ingredient.

9. The active ingredient according to one of the preceding claims, **characterized in that** it is present in liquid form.

10. A cosmetic use of a cosmetic active ingredient derived from the endosperm of the fruit of *Cocos nucifera* according to one of claims 1 to 9, or of a composition containing same, for topical application to the skin to brighten the skin.

11. A cosmetic use of an active ingredient derived from the endosperm of the fruit of *Cocos nucifera* according to one of claims 1 to 9, or of a composition containing same, for topical application to the skin for a regenerative effect on the skin.

12. A cosmetic use of an active ingredient derived from the endosperm of the fruit of *Cocos nucifera* according to one of claims 1 to 9, or of a composition containing same, for topical application to the skin to improve the quality of the cutaneous barrier function.

13. A cosmetic use of a cosmetic active ingredient derived from the endosperm of the fruit of *Cocos nucifera* according to one of claims 1 to 9, or of a composition containing same, for topical application to the skin to smooth and/or hydrate the skin.

14. A cosmetic use of an active ingredient derived from the endosperm of the fruit of *Cocos nucifera* according to one of claims 1 to 9, or of a composition containing same, for topical application to the skin to act on the OR2A4 and/or OR51B5 olfactory receptors present in keratinocytes.

15. A cosmetic use of an active ingredient derived from the endosperm of the fruit of *Cocos nucifera* according to one of claims 1 to 9, or of a composition containing same, for topical application to the skin to act on the TAS2R1 taste receptors present in keratinocytes.

16. A non-therapeutic cosmetic use of a cosmetic active ingredient derived from the endosperm of the fruit of *Cocos nucifera* according to one of claims 1 to 9, or of a composition containing same, for topical application to healthy skin to improve keratinocyte migration.

17. A non-therapeutic cosmetic use of a cosmetic active ingredient derived from the endosperm of the fruit of *Cocos nucifera* according to one of claims 1 to 9, or of a composition containing same, for topical application to the skin to enhance the sensory properties of the skin.

18. A cosmetic composition comprising at least 0.5% by weight of an active ingredient according to one of claims 1 to 9.

19. The cosmetic composition according to claim 18, **characterized in that** it is in the form of a gel, emulsion, or cream.

20. A cosmetic use of a composition according to one of claims 18 or 19 in a cosmetic method for treating skin for a regenerative effect on the skin, **characterized in that** said method consists of the topical application of said composition to the skin.

21. A cosmetic method for treating healthy skin for a moisturizing and/or smoothing effect and/or to enhance the sensory properties of the skin, consisting of the topical application of a composition according to one of claims 18 or 19 to the skin.
